# EUROPEAN PATENT SPECIFICATION

(11) **EP 3 629 952 B1**
(45) Date of publication and mention of the grant of the patent: **12.01.2022**
(21) Application number: 18730577.6
(22) Date of filing: 21.05.2018
(51) Int. Cl.: A61B 17/32, A61B 18/00, A61B 18/12, A61B 18/14, A61B 17/00, A61B 17/29, A61B 90/00

(54) **COMBINATION ULTRASONIC AND ELECTROSURGICAL INSTRUMENT HAVING ULTRASONIC WAVEGUIDE WITH DISTAL OVERMOLD MEMBER**
KOMBINATION EINES ULTRASCHALL- UND ELEKTROCHIRURGISCHEN INSTRUMENTES MIT EINEM ULTRASCHALL-WELLENLEITER UND EINEM DISTALEN UMFORMENDEN ELEMENT
COMBINAISON D'UN INSTRUMENT D'ULTRASON ET D'ÉLECTROCHIRURGIE AVEC UN GUIDE D'ONDES ET UN ÉLÉMENT ENTOURNANT DISTAL

(30) Priority: 22.05.2017 US 201762509351 P; 01.05.2018 US 201815967759
(43) Date of publication of application: 08.04.2020
(73) Proprietor: Ethicon LLC, 00969 Guaynabo (PR)
(72) Inventor: ESTERA, Frederick, L., Cincinnati OH 45242 (US); KEENAN, Michael, A., Cincinnati OH 45242 (US); DAVIS, Craig, T., Cincinnati OH 45242 (US); WEISENBURGH, William, B., II, Cincinnati OH 45242 (US); LESKO, Jason, R., Cincinnati OH 45242 (US)
(74) Representative: Carpmaels & Ransford LLP
(86) International application number: PCT/US2018/033615
(87) International publication number: WO 2019/013867

(56) References cited:
- EP-A1- 0 908 155
- US-A1- 2015 245 850
- US-B2- 9 107 690

## Description

This application claims the benefit of U.S. Provisional App. No. 62/509,351, entitled "Ultrasonic Instrument With Electrosurgical Features," filed May 22, 2017.

### BACKGROUND

Ultrasonic surgical instruments utilize ultrasonic energy for both precise cutting and controlled coagulation of tissue. The ultrasonic energy cuts and coagulates by vibrating a blade in contact with the tissue. Vibrating at frequencies of approximately 50 kilohertz (kHz), for example, the ultrasonic blade denatures protein in the tissue to form a sticky coagulum. Pressure exerted on the tissue with the blade surface collapses blood vessels and allows the coagulum to form a hemostatic seal. The precision of cutting and coagulation may be controlled by the surgeon's technique and adjusting the power level, blade edge, tissue traction, and blade pressure, for example.

Examples of ultrasonic surgical devices include the HARMONIC ACE^{®} Ultrasonic Shears, the HARMONIC WAVE^{®} Ultrasonic Shears, the HARMONIC FOCUS^{®} Ultrasonic Shears, and the HARMONIC SYNERGY^{®} Ultrasonic Blades, all by Ethicon Endo-Surgery, Inc. of Cincinnati, Ohio. Further examples of such devices and related concepts are disclosed in U.S. Pat. No. 5,322,055, entitled "Clamp Coagulator/Cutting System for Ultrasonic Surgical Instruments," issued Jun. 21, 1994; U.S. Pat. No. 5,873,873, entitled "Ultrasonic Clamp Coagulator Apparatus Having Improved Clamp Mechanism," issued Feb. 23, 1999; U.S. Pat No. 5,980,510, entitled "Ultrasonic Clamp Coagulator Apparatus Having Improved Clamp Arm Pivot Mount," issued Nov. 9, 1999; U.S. Pat. No. 6,283,981, entitled "Method of Balancing Asymmetric Ultrasonic Surgical Blades," issued Sep. 4, 2001; U.S. Pat. No. 6,309,400, entitled "Curved Ultrasonic Blade having a Trapezoidal Cross Section," issued Oct. 30, 2001; U.S. Pat No. 6,325,811, entitled "Blades with Functional Balance Asymmetries for use with Ultrasonic Surgical Instruments," issued Dec. 4, 2001; U.S. Pat No. 6,423,082, entitled "Ultrasonic Surgical Blade with Improved Cutting and Coagulation Features," issued Jul. 23, 2002; U.S. Pat. No. 6,773,444, entitled "Blades with Functional Balance Asymmetries for Use with Ultrasonic Surgical Instruments," issued Aug. 10, 2004; U.S. Pat No. 6,783,524, entitled "Robotic Surgical Tool with Ultrasound Cauterizing and Cutting Instrument," issued Aug. 31, 2004; U.S. Pat. No. 8,057,498, entitled "Ultrasonic Surgical Instrument Blades," issued Nov. 15, 2011; U.S. Pat. No. 8,461,744, entitled "Rotating Transducer Mount for Ultrasonic Surgical Instruments," issued Jun. 11, 2013; U.S. Pat. No. 8,591,536, entitled "Ultrasonic Surgical Instrument Blades," issued Nov. 26, 2013; U.S. Pat No. 8,623,027, entitled "Ergonomic Surgical Instruments," issued Jan. 7, 2014; U.S. Pat. No. 9,095,367, entitled "Flexible Harmonic Waveguides/Blades for Surgical Instruments," issued Aug. 4, 2015; and U.S. Pub. No. 2016/0022305, entitled "Ultrasonic Blade Overmold," published Jan. 28, 2016.

Electrosurgical instruments utilize electrical energy for sealing tissue, and generally include a distally mounted end effector that can be configured for bipolar or monopolar operation. During bipolar operation, electrical current is provided through the tissue by active and return electrodes of the end effector. During monopolar operation, current is provided through the tissue by an active electrode of the end effector and a return electrode (*e.g.,* a grounding pad) separately located on a patient's body. Heat generated by the current flowing through the tissue may form hemostatic seals within the tissue and/or between tissues, and thus may be particularly useful for sealing blood vessels, for example. The end effector of an electrosurgical device may also include a cutting member that is movable relative to the tissue and the electrodes to transect the tissue.

Electrical energy applied by an electrosurgical device can be transmitted to the instrument by a generator coupled with the instrument The electrical energy may be in the form of radio frequency ("RF") energy, which is a form of electrical energy generally in the frequency range of approximately 300 kilohertz (kHz) to 1 megahertz (MHz). In use, an electrosurgical device can transmit lower frequency RF energy through tissue, which causes ionic agitation, or friction, in effect resistive heating, thereby increasing the temperature of the tissue. Because a sharp boundary is created between the affected tissue and the surrounding tissue, surgeons can operate with a high level of precision and control, without sacrificing un-targeted adjacent tissue. The low operating temperatures of RF energy may be useful for removing, shrinking, or sculpting soft tissue while simultaneously sealing blood vessels. RF energy works particularly well on connective tissue, which is primarily comprised of collagen and shrinks when contacted by heat.

An example of an RF electrosurgical device is the ENSEAL^{®} Tissue Sealing Device by Ethicon Endo-Surgery, Inc., of Cincinnati, Ohio. Further examples of electrosurgical devices and related concepts are disclosed in U.S. Pat. No. 6,500,176 entitled "Electrosurgical Systems and Techniques for Sealing Tissue," issued Dec. 31, 2002; U.S. Pat. No. 7,112,201 entitled "Electrosurgical Instrument and Method of Use," issued Sep. 26, 2006; U.S. Pat. No. 7,125,409, entitled "Electrosurgical Working End for Controlled Energy Delivery," issued Oct 24, 2006; Pat. No. 7,169,146 entitled "Electrosurgical Probe and Method of Use," issued Jan. 30, 2007; U.S. Pat No. 7,186,253, entitled "Electrosurgical Jaw Structure for Controlled Energy Delivery," issued Mar. 6, 2007; U.S. Pat No. 7,189,233, entitled "Electrosurgical Instrument," issued Mar. 13, 2007; U.S. Pat. No. 7,220,951, entitled "Surgical Sealing Surfaces and Methods of Use," issued May 22, 2007; U.S. Pat. No. 7,309,849, entitled "Polymer Compositions Exhibiting a PTC Property and Methods of Fabrication," issued Dec. 18, 2007; U.S. Pat. No. 7,311,709, entitled "Electrosurgical Instrument and Method of Use," issued Dec. 25, 2007; U.S. Pat No. 7,354,440, entitled "Electrosurgical Instrument and Method of Use," issued Apr. 8, 2008; U.S. Pat. No. 7,381,209, entitled "Electrosurgical Instrument," issued Jun. 3, 2008.

Additional examples of electrosurgical devices and related concepts are disclosed in U.S. Pat No. 8,939,974, entitled "Surgical Instrument Comprising First and Second Drive Systems Actuatable by a Common Trigger Mechanism," issued Jan. 27, 2015; U.S. Pat No. 9,161,803, entitled "Motor Driven Electrosurgical Device with Mechanical and Electrical Feedback," issued Oct 20, 2015; U.S. Pub. No. 2012/0078243, entitled "Control Features for Articulating Surgical Device," published Mar. 29, 2012; U.S. Pat. No. 9,402,682, entitled "Articulation Joint Features for Articulating Surgical Device," issued Aug. 2, 2016; U.S. Pat. No. 9,089,327, entitled "Surgical Instrument with Multi-Phase Trigger Bias," issued July 28, 2015; U.S. Pat. No. 9,545,253, entitled "Surgical Instrument with Contained Dual Helix Actuator Assembly," issued Jan. 17, 2017; and U.S. Pat. No. 9,572,622, entitled "Bipolar Electrosurgical Features for Targeted Hemostasis," issued Feb. 21, 2017.

Some instruments may provide ultrasonic and RF energy treatment capabilities through a single surgical device. Examples of such devices and related methods and concepts are disclosed in U.S. Pat No. 8,663,220, entitled "Ultrasonic Surgical Instruments," issued March 4, 2014; U.S. Pub. No. 2015/0141981, entitled "Ultrasonic Surgical Instrument with Electrosurgical Feature," published May 21, 2015; and U.S. Pub. No. 2017/0000541, entitled "Surgical Instrument with User Adaptable Techniques," published Jan. 5, 2017.

While various types of ultrasonic surgical instruments and electrosurgical instruments, including combination ultrasonic-electrosurgical instruments, have been made and used, it is believed that no one prior to the inventor(s) has made or used the invention described in the appended claims.

In US2015245850A1 there is described an apparatus comprising a body, an ultrasonic transducer assembly, a shaft assembly, and a coupling assembly.

In US9107690B2 there is described a battery assembly for use with a surgical device having a battery terminal and operational parameters that includes a housing having a shape formed to removably connect with the terminal.

In EP0908155A1 there is described an ultrasonic surgical clamp coagulator apparatus configured to effect cutting, coagulation, and clamping of tissue by cooperation of a clamping mechanism of the apparatus with an associated ultrasonic end-effector. The ultrasonic waveguide of the apparatus is preferably provided with an improved support member at the distal-most node thereof, which support member desirably functions to resist bending moments created in the waveguide, attendant to operation of the clamping mechanism, while sealing the region adjacent to the waveguide.

### DETAILED DESCRIPTION

The invention is defined in claim 1. Further embodiments are defined in the dependent claims.

The following description of certain examples of the disclosure should not be used to limit the scope of the present disclosure. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the descriptions should be regarded as illustrative in nature and not restrictive.

For clarity of disclosure, the terms "proximal" and "distal" are defined herein relative to a surgeon, or other operator, grasping a surgical instrument having a distal surgical end effector. The term "proximal" refers to the position of an element arranged closer to the surgeon, and the term "distal" refers to the position of an element arranged closer to the surgical end effector of the surgical instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

### I. Exemplary Surgical System

Hereby disclosed is an exemplary surgical system including a generator and a surgical instrument. Surgical instrument is operatively coupled with the generator via power cable. As described in greater detail below, generator is operable to power surgical instrument to deliver ultrasonic energy for cutting tissue, and electrosurgical bipolar RF energy (i.e., therapeutic levels of RF energy) for sealing tissue. In exemplary configurations, generator is configured to power surgical instrument to deliver ultrasonic energy and electrosurgical bipolar RF energy simultaneously.

### A. Overview of Exemplary Surgical Instrument with Ultrasonic and Electrosurgical Features

Surgical instrument of the present example comprises a handle assembly, a shaft assembly extending distally from the handle assembly, and an end effector arranged at a distal end of the shaft assembly. Handle assembly comprises a body including a pistol grip and energy control buttons configured to be manipulated by a surgeon. A trigger is coupled to a lower portion of body and is pivotable toward and away from pistol grip to selectively actuate end effector, as described in greater detail below. In other suitable variations of surgical instrument, handle assembly may comprise a scissor grip configuration, for example. As described in greater detail below, an ultrasonic transducer is housed internally within and supported by body. In other configurations, ultrasonic transducer may be provided externally of body.

End effector includes an ultrasonic blade and a clamp arm configured to selectively pivot toward and away from ultrasonic blade, for clamping tissue therebetween. Ultrasonic blade is acoustically coupled with ultrasonic transducer, which is configured to drive (i.e., vibrate) ultrasonic blade at ultrasonic frequencies for cutting and/or sealing tissue positioned in contact with ultrasonic blade. Clamp arm is operatively coupled with trigger such that clamp arm is configured to pivot toward ultrasonic blade, to a closed position, in response to pivoting of trigger toward pistol grip. Further, clamp arm is configured to pivot away from ultrasonic blade, to an open position , in response to pivoting of trigger away from pistol grip. Various suitable ways in which clamp arm may be coupled with trigger will be apparent to those of ordinary skill in the art in view of the teachings provided herein. In some versions, one or more resilient members may be incorporated to bias clamp arm and/or trigger toward the open position.

A clamp pad is secured to and extends distally along a clamping side of clamp arm, facing ultrasonic blade. Clamp pad is configured to engage and clamp tissue against a corresponding tissue treatment portion of ultrasonic blade when clamp arm is actuated to its closed position. At least a clamping-side of clamp arm provides a first electrode, referred to herein as clamp arm electrode. Additionally, at least a clamping-side of ultrasonic blade provides a second electrode, referred to herein as a blade electrode. As described in greater detail below, electrodes are configured to apply electrosurgical bipolar RF energy, provided by generator, to tissue electrically coupled with electrodes. Clamp arm electrode may serve as an active electrode while blade electrode serves as a return electrode, or vice-versa. Surgical instrument may be configured to apply the electrosurgical bipolar RF energy through electrodes while vibrating ultrasonic blade at an ultrasonic frequency, before vibrating ultrasonic blade at an ultrasonic frequency, and/or after vibrating ultrasonic blade at an ultrasonic frequency.

Shaft assembly extends along a longitudinal axis and includes an outer tube, an inner tube received within outer tube, and an ultrasonic waveguide supported within inner tube. Clamp arm is coupled to distal ends of inner and outer tubes. In particular, clamp arm includes a pair of proximally extending clevis arms that receive therebetween and pivotably couple to a distal end of inner tube with a pivot pin received within through bores formed in clevis arms and distal end of inner tube. First and second clevis fingers depend downwardly from clevis arms and pivotably couple to a distal end of outer tube. Specifically, each clevis finger includes a protrusion that is rotatably received within a corresponding opening formed in a sidewall of distal end of outer tube.

In the present example, inner tube is longitudinally fixed relative to handle assembly, and outer tube is configured to translate relative to inner tube and handle assembly, along the longitudinal axis of shaft assembly. As outer tube translates distally, clamp arm pivots about pivot pin toward its open position. As outer tube translates proximally, clamp arm pivots in an opposite direction toward its closed position. A proximal end of outer tube is operatively coupled with trigger, for example via a linkage assembly, such that actuation of trigger causes translation of outer tube relative to inner tube, thereby opening or closing clamp arm. In other suitable configurations not shown herein, outer tube may be longitudinally fixed and inner tube may be configured to translate for moving clamp arm between its open and closed positions.

Shaft assembly and end effector are configured to rotate together about the longitudinal axis, relative to handle assembly. A retaining pin extends transversely through proximal portions of outer tube, inner tube, and waveguide to thereby couple these components rotationally relative to one another. In the present example, a rotation knob is provided at a proximal end portion of shaft assembly to facilitate rotation of shaft assembly, and end effector, relative to handle assembly. Rotation knob is secured rotationally to shaft assembly with retaining pin, which extends through a proximal collar of rotation knob. It will be appreciated that in other suitable configurations, rotation knob may be omitted or substituted with alternative rotational actuation structures.

Ultrasonic waveguide is acoustically coupled at its proximal end with ultrasonic transducer, for example by a threaded connection, and at its distal end with ultrasonic blade. Ultrasonic blade is shown formed integrally with waveguide such that blade extends distally, directly from the distal end of waveguide. In this manner, waveguide acoustically couples ultrasonic transducer with ultrasonic blade, and functions to communicate ultrasonic mechanical vibrations from transducer to blade. Accordingly, ultrasonic transducer, waveguide, and ultrasonic blade together define acoustic assembly. During use, ultrasonic blade may be positioned in direct contact with tissue, with or without assistive clamping force provided by clamp arm, to impart ultrasonic vibrational energy to the tissue and thereby cut and/or seal the tissue. For example, blade may cut through tissue clamped between clamp arm and a first treatment side of blade, or blade may cut through tissue positioned in contact with an oppositely disposed second treatment side of blade, for example during a "back-cutting" movement. In some variations, waveguide may amplify the ultrasonic vibrations delivered to blade. Further, waveguide may include various features operable to control the gain of the vibrations, and/or features suitable to tune waveguide to a selected resonant frequency. Additional exemplary features of ultrasonic blade and waveguide are described in greater detail below.

Waveguide is supported within inner tube by a plurality of nodal support elements positioned along a length of waveguide. Specifically, nodal support elements are positioned longitudinally along waveguide at locations corresponding to acoustic nodes defined by the resonant ultrasonic vibrations communicated through waveguide. Nodal support elements may provide structural support to waveguide, and acoustic isolation between waveguide and inner and outer tubes of shaft assembly. In exemplary variations, nodal support elements may comprise o-rings. Waveguide is supported at its distal-most acoustic node by a nodal support element in the form of an overmold member described in greater detail below. Waveguide is secured longitudinally and rotationally within shaft assembly by retaining pin, which passes through a transverse through-bore formed at a proximally arranged acoustic node of waveguide, such as the proximal-most acoustic node, for example.

In the present example, a distal tip of ultrasonic blade is located at a position corresponding to an anti-node associated with the resonant ultrasonic vibrations communicated through waveguide. Such a configuration enables the acoustic assembly of instrument to be tuned to a preferred resonant frequency fₒ when ultrasonic blade is not loaded by tissue. When ultrasonic transducer is energized by generator to transmit mechanical vibrations through waveguide to blade, distal tip of blade is caused to oscillate longitudinally in the range of approximately 20 to 120 microns peak-to-peak, for example, and in some instances in the range of approximately 20 to 50 microns, at a predetermined vibratory frequency fₒ of approximately 50 kHz, for example. When ultrasonic blade is positioned in contact with tissue, the ultrasonic oscillation of blade may simultaneously sever the tissue and denature the proteins in adjacent tissue cells, thereby providing a coagulative effect with minimal thermal spread.

Distal end of inner tube may be offset radially outwardly relative to a remaining proximal portion of inner tube. This configuration enables pivot pin bore, which receives clamp arm pivot pin, to be spaced further away from the longitudinal axis of shaft assembly than if distal end where formed flush with the remaining proximal portion of inner tube. Advantageously, this provides increased clearance between proximal portions of clamp arm electrode and blade electrode, thereby mitigating risk of undesired "shorting" between electrodes and their corresponding active and return electrical paths, for example during back-cutting when ultrasonic blade flexes toward clamp arm and pivot pin in response to normal force exerted on blade by tissue. In other words, when ultrasonic blade is used in a back-cutting operation, ultrasonic blade may tend to deflect slightly away from the longitudinal axis of shaft assembly, toward pin. By having pivot pin bore spaced further away from the longitudinal axis than pivot pin bore otherwise would be in the absence of the radial offset provided by distal end of the present example, distal end provides additional lateral clearance between pivot pin and ultrasonic blade, thereby reducing or eliminating the risk of contact between ultrasonic blade and pivot pin when ultrasonic blade deflects laterally during back-cutting operations. In addition to preventing electrical short circuits that would otherwise result from contact between ultrasonic blade and pivot pin when end effector is activated to apply RF electrosurgical energy, the additional clearance prevents mechanical damage that might otherwise result from contact between ultrasonic blade and pivot pin when ultrasonic blade is vibrating ultrasonically.

### B. Exemplary Overmold Member for Ultrasonic Waveguide

Hereby disclosed are the additional details of overmold member. As described above, overmold member encircles waveguide at a distal-most acoustic node thereof, thereby supporting waveguide within inner tube and defining a distal-most waveguide support location. Waveguide includes an annular nodal flange at its distal-most acoustic node, and overmold member encircles nodal flange. Ultrasonic blade is integrally joined to waveguide at distal nodal flange and extends distally therefrom.

Overmold member includes a load bearing portion and an integrally formed sealing portion extending proximally from load bearing portion. As described below, each of load bearing support portion and sealing portion is configured to engage an inner surface of inner tube. Load bearing support portion includes an inner annular groove configured to receive distal nodal flange of waveguide in sealing engagement, such that load bearing support portion aligns with and encircles distal nodal flange while sealing portion extends proximally of distal nodal flange.

Load bearing support portion of overmold member includes a plurality of deforming elements spaced circumferentially about its exterior. Deforming elements define a maximum outer diameter of load bearing portion that is greater than an inner diameter of inner tube. Accordingly, deforming elements are configured to resiliently deform against the inner surface of inner tube so as to engage inner tube with an interference fit Circumferential spacing between deforming elements enables elements to deform in a circumferential direction along the inner surface of inner tube. In this manner, load bearing support portion is configured to support waveguide in coaxial alignment with the longitudinal axis of shaft assembly, and mitigate radial displacement of distal nodal flange relative to the longitudinal axis when waveguide is driven with ultrasonic energy, as described above. Advantageously, this prevents unwanted direct contact between ultrasonic blade and clamp arm, or clamp arm pivot pin, which could otherwise cause mechanical failure of blade and/or electrical shorting of an RF electrical circuit of surgical instrument Overmold member may be formed of any material or combination of materials suitable to acoustically isolate distal nodal flange relative to inner tube. For instance, at least deforming elements and sealing portion may be formed of a resiliently deformable polymeric material, such as silicone, for example.

Each deforming element is shown in the form of a rounded protrusion, or bump, integrally formed with load bearing support portion and projecting radially outwardly from an outer surface thereof, and extending axially. Load bearing support portion includes four deforming elements arranged with uniform circumferential spacing. In other configurations, any suitable quantity and circumferential spacing of deforming elements may be provided. A distal end of load bearing support portion may taper from deforming elements toward ultrasonic blade.

Sealing portion of overmold member is spaced proximally from load bearing support portion by an outer annular groove. Sealing portion includes an annular outer sealing edge configured as a wiper seal that resiliently deforms against, and thereby establishes a liquid-tight seal with, a full inner circumference of the inner surface of inner tube. As shown, an axial dimension of sealing edge is substantially less than an axial dimension of deforming elements. Accordingly, while deforming elements are configured to provide structural support to waveguide, sealing edge is configured to maintain a liquid-tight seal against inner tube to prevent proximal ingress of body fluids and tissue into shaft assembly along waveguide. Such ingress could yield undesirable reduction of ultrasonic energy delivery from waveguide to ultrasonic blade, and/or electrical coupling of waveguide to inner tube, which could result in shorting of the RF electrical circuit of surgical instrument. Outer sealing edge defines a maximum outer diameter of sealing portion, which may be equal to, slightly less than, or slightly greater than the maximum outer diameter of load bearing portion defined by deforming elements.

### C. Exemplary Alternative Overmold Member for Ultrasonic Waveguide

Hereby disclosed is another exemplary overmold member suitable for use with surgical instrument in place of overmold member. Overmold member is similar to overmold member described above except as otherwise described below. Similar to overmold member, overmold member encircles nodal flange at the distal-most acoustic node of waveguide to thereby support waveguide coaxially within inner tube. Unlike overmold member, an exterior of overmold member includes an annular rim that engages an inner surface of inner tube with an interference fit so as to function as both a load bearing portion and a sealing portion, similar to load bearing portion and sealing portion of overmold member described above.

Annular rim of overmold member of the present example is positioned at a medial portion of overmold member such that annular rim is aligned with distal nodal flange of waveguide. Annular rim extends continuously about a full circumference of overmold member such that rim is configured to establish a continuous seal with the inner surface of inner tube. Moreover, annular rim extends radially outwardly to define a maximum outer diameter of overmold member that provides a degree of interference with inner tube sufficient to provide both mechanical support and annular sealing about the full circumference of waveguide. In some examples, annular rim may provide a higher degree of interference with inner tube than deforming elements of overmold member. However, similar to deforming elements, at least annular rim of overmold member may be formed of a resiliently deformable polymeric material, such as silicone, for example. Though not shown, overmold member may include one or more additional annular features arranged proximally or distally of annular rim and configured to sealingly engage the inner surface of inner tube, for instance similar to annular sealing edge described above.

Overmold member of the present example further includes a proximal tapered portion that extends proximally from annular rim, a distal tapered portion that extends distally from annular rim, and a distal flap that extends distally from a distal end of distal tapered portion. Proximal tapered portion tapers inwardly from annular rim in a proximal direction, and distal tapered portion tapers inwardly from annular rim in a distal direction. Proximal and distal tapered portions may be formed with similar axial lengths and taper angles and are configured to facilitate axial assembly of inner tube over waveguide and overmold member. Distal flap overlaps a proximal end of ultrasonic blade and is configured to create an annular seal between overmold member and the corresponding portion of waveguide and ultrasonic blade covered by distal flap. It will be appreciated that various other versions of overmold member may be employed that incorporate one or more features from each of the overmold members to provide annular sealing and mechanical support about the circumference of waveguide within inner tube.

### III. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued Aug. 11, 1998; U.S. Pat No. 5,817,084, entitled "Remote Center Positioning Device with Flexible Drive," issued Oct 6, 1998; U.S. Pat No. 5,878,193, entitled "Automated Endoscope System for Optimal Positioning," issued March 2, 1999; U.S. Pat. No. 6,231,565, entitled "Robotic Arm DLUS for Performing Surgical Tasks," issued May 15, 2001; U.S. Pat No. 6,783,524, entitled "Robotic Surgical Tool with Ultrasound Cauterizing and Cutting Instrument," issued Aug. 31, 2004; U.S. Pat. No. 6,364,888, entitled "Alignment of Master and Slave in a Minimally Invasive Surgical Apparatus," issued April 2, 2002; U.S. Pat. No. 7,524,320, entitled "Mechanical Actuator Interface System for Robotic Surgical Tools," issued April 28, 2009; U.S. Pat. No. 7,691,098, entitled "Platform Link Wrist Mechanism," issued April 6, 2010; U.S. Pat No. 7,806,891, entitled "Repositioning and Reorientation of Master/Slave Relationship in Minimally Invasive Telesurgery," issued Oct 5, 2010; U.S. Pat No. 8,844,789, entitled "Automated End Effector Component Reloading System for Use with a Robotic System," issued Sept 30, 2014; U.S. Pat No. 8,820,605, entitled "Robotically-Controlled Surgical Instruments," issued Sept 2, 2014; U.S. Pat No. 8,616,431, entitled "Shiftable Drive Interface for Robotically-Controlled Surgical Tool," issued Dec. 31, 2013; U.S. Pat. No. 8,573,461, entitled "Surgical Stapling Instruments with Cam-Driven Staple Deployment Arrangements," issued Nov. 5, 2013; U.S. Pat. No. 8,602,288, entitled "Robotically-Controlled Motorized Surgical End Effector System with Rotary Actuated Closure Systems Having Variable Actuation Speeds," issued Dec. 10, 2013; U.S. Pat. No. 9,301,759, entitled "Robotically-Controlled Surgical Instrument with Selectively Articulatable End Effector," issued April 5, 2016; U.S. Pat. No. 8,783,541, entitled "Robotically-Controlled Surgical End Effector System," issued July 22, 2014; U.S. Pat No. 8,479,969, entitled "Drive Interface for Operably Coupling a Manipulatable Surgical Tool to a Robot," issued July 9, 2013; U.S. Pat. Pub. No. 8,800,838, entitled "Robotically-Controlled Cable-Based Surgical End Effectors," issued Aug. 12, 2014; and/or U.S. Pat. No. 8,573,465, entitled "Robotically-Controlled Surgical End Effector System with Rotary Actuated Closure Systems," issued Nov. 5, 2013.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present disclosure.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present disclosure, further adaptations of the methods and systems described herein may be accomplished by appropriate modifications by one of ordinary skill in the art without departing from the scope of the present disclosure. Several of such potential modifications have been mentioned, and others will be apparent to those skilled in the art For instance, the examples, embodiments, geometries, materials, dimensions, ratios, steps, and the like discussed above are illustrative and are not required. Accordingly, the scope of the present invention should be considered in terms of the following claims.

## Claims

1. A surgical instrument comprising:
(a) an ultrasonic transducer;
(b) a shaft extending distally relative to the ultrasonic transducer along a longitudinal shaft axis;
(c) a waveguide acoustically coupled with the ultrasonic transducer and extending distally through the shaft;
(d) an end effector arranged at a distal end of the shaft, wherein the end effector includes an ultrasonic blade acoustically coupled with the waveguide, wherein the ultrasonic transducer is operable to drive the waveguide and the ultrasonic blade with ultrasonic energy; and
(e) a nodal support element arranged within a distal portion of the shaft, wherein the nodal support element encircles the waveguide at a distal-most acoustic node thereof, wherein the nodal support element comprises:
(i) a support portion aligned with the distal-most acoustic node, wherein the support portion is configured to engage an inner surface of the shaft and thereby support the waveguide in coaxial alignment with the shaft axis, wherein the support portion includes a plurality of deformable elements spaced circumferentially about an exterior thereof, wherein the deformable elements are configured to deform against the inner surface of the shaft; and
(ii) a sealing portion extending axially from the support portion, wherein the sealing portion is configured to sealingly engage the inner surface of the shaft and thereby prevent proximal ingress of fluid through the shaft.

2. The surgical instrument of claim 1, wherein the nodal support element comprises an overmold member.

3. The surgical instrument of claim 1, wherein the support portion has a maximum outer diameter that is greater than an inner diameter of the shaft, wherein the support portion is configured to engage the inner surface of the shaft with an interference fit.

4. The surgical instrument of claim 1, wherein the deformable elements are resiliently deformable.

5. The surgical instrument of claim 1, wherein the deformable elements are arranged with uniform circumferential spacing.

6. The surgical instrument of claim 1, wherein the plurality of deformable elements comprises at least four deformable elements.

7. The surgical instrument of claim 1, wherein the sealing portion extends proximally from the support portion.

8. The surgical instrument of claim 1, wherein the sealing portion includes an annular sealing edge configured to sealingly engage the inner surface of the shaft.

9. The surgical instrument of claim 8, wherein the annular sealing edge is spaced axially from the support portion by an annular groove formed in an exterior of the nodal support element.

10. The surgical instrument of claim 1, wherein the support portion has an axial length greater than an axial length of the sealing portion.

11. The surgical instrument of claim 1, wherein the waveguide includes a nodal flange at the distal-most acoustic node, wherein the ultrasonic blade integrally joins with the waveguide at the nodal flange, wherein an interior of the support portion includes an annular groove configured to receive the nodal flange.

12. The surgical instrument of claim 1, wherein the shaft comprises an inner tube and an outer tube, wherein the nodal support element engages an inner surface of the inner tube.

13. The surgical instrument of claim 1, wherein the end effector further comprises an RF electrode, wherein the RF electrode is operable to seal tissue with RF energy.

14. The surgical instrument of claim 1, wherein the end effector further comprises a clamp arm movable relative to the ultrasonic blade to clamp tissue therebetween, wherein the clamp arm provides a first RF electrode, wherein the ultrasonic blade provides a second RF electrode, wherein the first and second RF electrodes are operable to seal tissue with bipolar RF energy.

## Patentansprüche

1. Chirurgisches Instrument, umfassend:
(a) einen Ultraschallwandler;
(b) einen Schaft, der sich relativ zu dem Ultraschallwandler entlang einer längsgerichteten Achse des Schafts distal erstreckt;
(c) einen Wellenleiter, der akustisch mit dem Ultraschallwandler gekoppelt ist und sich distal durch den Schaft hindurch erstreckt;
(d) einen Endeffektor, der an einem distalen Ende des Schafts angeordnet ist, wobei der Endeffektor eine Ultraschallklinge einschließt, die akustisch mit dem Wellenleiter gekoppelt ist, wobei der Ultraschallwandler funktional ist, um den Wellenleiter und die Ultraschallklinge mit Ultraschallenergie anzutreiben; und
(e) ein Knotenstützelement, das innerhalb eines distalen Anteils des Schafts angeordnet ist, wobei das Knotenstützelement den Wellenleiter an einem am weitesten distal liegenden akustischen Knoten desselben umschließt, wobei das Knotenstützelement umfasst:
(i) einen Stützanteil, der mit dem am weitesten distal liegenden akustischen Knoten ausgerichtet ist, wobei der Stützanteil ausgestaltet ist, um in Eingriff mit einer Innenoberfläche des Schafts zu kommen und dadurch den Wellenleiter in koaxialer Ausrichtung mit der Schaftachse zu stützen, wobei der Stützanteil eine Vielzahl verformbarer Elemente einschließt, die umlaufend um einen Außenbereich davon beabstandet sind, wobei die verformbaren Elemente ausgestaltet sind, um sich gegen die Innenoberfläche des Schafts zu verformen; und
(ii) einen Dichtungsanteil, der sich axial von dem Stützanteil erstreckt, wobei der Dichtungsanteil ausgestaltet ist, um dichtend in Eingriff mit der Innenoberfläche des Schafts zu kommen und dadurch proximales Eindringen von Fluid durch den Schaft hindurch zu verhindern.

2. Chirurgisches Instrument nach Anspruch 1, wobei das Knotenstützelement ein umspritztes Teil umfasst.

3. Chirurgisches Instrument nach Anspruch 1, wobei der Stützanteil einen maximalen Außendurchmesser aufweist, der größer als ein Innendurchmesser des Schafts ist, wobei der Stützanteil ausgestaltet ist, um mit einer Presspassung in Eingriff mit der Innenoberfläche des Schafts zu kommen.

4. Chirurgisches Instrument nach Anspruch 1, wobei die verformbaren Elemente elastisch verformbar sind.

5. Chirurgisches Instrument nach Anspruch 1, wobei die verformbaren Elemente mit in Umlaufrichtung gleichförmigem Abstand angeordnet sind.

6. Chirurgisches Instrument nach Anspruch 1, wobei die Vielzahl verformbarer Elemente mindestens vier verformbare Elemente umfasst.

7. Chirurgisches Instrument nach Anspruch 1, wobei der Dichtungsanteil sich proximal des Stützanteils erstreckt.

8. Chirurgisches Instrument nach Anspruch 1, wobei der Dichtungsanteil einen kranzförmigen Dichtungsrand einschließt, der ausgestaltet ist, um dichtend in Eingriff mit der Innenoberfläche des Schafts zu kommen.

9. Chirurgisches Instrument nach Anspruch 8, wobei der kranzförmige Dichtungsrand axial durch eine kranzförmige Nut, die in einem Außenbereich des Knotenstützelements gebildet ist, von dem Stützanteil beabstandet ist.

10. Chirurgisches Instrument nach Anspruch 1, wobei der Stützanteil eine axiale Länge aufweist, die größer als eine axiale Länge des Dichtungsanteils ist.

11. Chirurgisches Instrument nach Anspruch 1, wobei der Wellenleiter einen Knotenflansch an dem am weitesten distal liegenden akustischen Knoten einschließt, wobei die Ultraschallklinge integral an dem Knotenflansch an den Wellenleiter angefügt ist, wobei ein Innenbereich des Stützanteils eine kranzförmige Nut einschließt, die ausgestaltet ist, um den Knotenflansch aufzunehmen.

12. Chirurgisches Instrument nach Anspruch 1, wobei der Schaft ein Innenrohr und ein Außenrohr umfasst, wobei das Knotenstützelement in Eingriff mit einer Innenoberfläche des Innenrohrs kommt.

13. Chirurgisches Instrument nach Anspruch 1, wobei der Endeffektor des Weiteren eine HF-Elektrode umfasst, wobei die HF-Elektrode funktional ist, um Gewebe mit HF-Energie zu versiegeln.

14. Chirurgisches Instrument nach Anspruch 1, wobei der Endeffektor des Weiteren einen Klemmarm umfasst, der relativ zu der Ultraschallklinge beweglich ist, um Gewebe dazwischen einzuklemmen, wobei der Klemmarm eine erste HF-Elektrode bereitstellt, wobei die Ultraschallklinge eine zweite HF-Elektrode bereitstellt, wobei die erste und die zweite HF-Elektrode funktional sind, um Gewebe mit bipolarer HF-Energie zu versiegeln.

## Revendications

1. Instrument chirurgical comprenant :
(a) un transducteur à ultrasons ;
(b) un arbre s'étendant de manière distale par rapport au transducteur à ultrasons le long d'un axe d'arbre longitudinal ;
(c) un guide d'ondes couplé acoustiquement au transducteur à ultrasons et s'étendant de manière distale à travers l'arbre ;
(d) un effecteur d'extrémité agencé à une extrémité distale de l'arbre, l'effecteur d'extrémité comprenant une lame ultrasonore couplée acoustiquement au guide d'ondes, le transducteur à ultrasons étant utilisable pour entraîner le guide d'ondes et la lame ultrasonore avec de l'énergie ultrasonore ; et
(e) un élément de support nodal agencé dans une partie distale de l'arbre, l'élément de support nodal encerclant le guide d'ondes au niveau d'un nœud acoustique le plus distal de celui-ci, l'élément de support nodal comprenant :
(i) une partie de support alignée avec le nœud acoustique le plus distal, la partie de support étant configurée pour venir en prise avec une surface intérieure de l'arbre et ainsi supporter le guide d'ondes en alignement coaxial avec l'axe de l'arbre, la partie de support comprenant une pluralité d'éléments déformables espacés circonférentiellement autour de son extérieur, les éléments déformables étant configurés pour se déformer contre la surface intérieure de l'arbre ; et
(ii) une partie d'étanchéité s'étendant axialement à partir de la partie de support, la partie d'étanchéité étant configurée pour venir en prise de manière étanche avec la surface intérieure de l'arbre et empêcher ainsi l'entrée proximale de fluide à travers l'arbre.

2. Instrument chirurgical selon la revendication 1, l'élément de support nodal comprenant un élément surmoulé.

3. Instrument chirurgical selon la revendication 1, la partie de support ayant un diamètre extérieur maximal qui est supérieur à un diamètre intérieur de la tige, la partie de support étant configurée pour venir en prise avec la surface intérieure de la tige avec un ajustement serré.

4. Instrument chirurgical selon la revendication 1, les éléments déformables étant élastiquement déformables.

5. Instrument chirurgical selon la revendication 1, les éléments déformables étant agencés avec un espacement circonférentiel uniforme.

6. Instrument chirurgical selon la revendication 1, la pluralité d'éléments déformables comprenant au moins quatre éléments déformables.

7. Instrument chirurgical selon la revendication 1, la partie d'étanchéité s'étendant de manière proximale depuis la partie de support.

8. Instrument chirurgical selon la revendication 1, la partie d'étanchéité comprenant un bord d'étanchéité annulaire configuré pour venir en prise de manière étanche avec la surface interne de l'arbre.

9. Instrument chirurgical selon la revendication 8, le bord d'étanchéité annulaire étant espacé axialement de la partie de support par une rainure annulaire formée dans un extérieur de l'élément de support nodal.

10. Instrument chirurgical selon la revendication 1, la partie de support ayant une longueur axiale supérieure à une longueur axiale de la partie d'étanchéité.

11. Instrument chirurgical selon la revendication 1, le guide d'ondes comprenant une bride nodale au niveau du nœud acoustique le plus distal, la lame ultrasonore se joignant de manière intégrée au guide d'ondes au niveau de la bride nodale, un intérieur de la partie de support comprenant une rainure annulaire configurée pour recevoir la bride nodale.

12. Instrument chirurgical selon la revendication 1, l'arbre comprenant un tube interne et un tube externe, l'élément de support nodal venant en prise avec une surface interne du tube interne.

13. Instrument chirurgical selon la revendication 1, l'effecteur d'extrémité comprenant en outre une électrode RF, l'électrode RF étant utilisable pour sceller le tissu avec de l'énergie RF.

14. Instrument chirurgical selon la revendication 1, l'effecteur d'extrémité comprenant en outre un bras de serrage mobile par rapport à la lame ultrasonore pour serrer le tissu entre eux, le bras de serrage fournissant une première électrode RF, la lame ultrasonore fournissant une seconde électrode RF, les première et seconde électrodes RF étant utilisables pour sceller le tissu avec une énergie RF bipolaire.
